# EUROPÄISCHE PATENTSCHRIFT

(11) **EP 0 697 220 B1**
(45) Veröffentlichungstag und Bekanntmachung des Hinweises auf die Patenterteilung: **04.09.2002**
(21) Anmeldenummer: 95109497.8
(22) Anmeldetag: 20.06.1995
(51) Int. Cl.: A61M 1/16

(54) **Flexible medizinische Verpackungseinheit für die Hämodialyse zur Herstellung eines Dialysierflüssigkeits-Konzentrats**
Flexible packaging unit for a dry concentrate for the preparation of a dialysate concentrate
Unité d'emballage flexible pour concentré en poudre destiné à la préparation d'un concentré de dialysat

(30) Priorität: 24.06.1994 DE 4422100
(43) Veröffentlichungstag der Anmeldung: 21.02.1996
(60) Teilanmeldung: 99103927.2
(73) Patentinhaber: Fresenius Medical Care Deutschland GmbH, 61352 Bad Homburg v.d.H. (DE)
(72) Erfinder: Mathieu, Bernd, Dr., D-66583 Spiesen (DE)
(74) Vertreter: Luderschmidt, Schüler & Partner GbR

(56) Entgegenhaltungen:
- EP-A- 0 469 487
- EP-A- 0 475 825
- EP-A- 0 575 970
- US-A- 4 386 634
- US-A- 5 385 564

## Beschreibung

Die Erfindung betrifft eine medizinische Verpackungseinheit für die Hämodialyse zur Herstellung eines Dialysierflüssigkeitskonzentrats mit einem Behälter, der ein einziges Anschlußstück und einen Innenraum aufweist, der mit einem pulverförmigen Salzkonzentrat mit einer für eine Dialysebehandlung ausreichenden Menge gefüllt ist.

Für die Hämodialyse muß eine Dialysierflüssigkeit bereitgestellt werden, deren Elektrolytzusammensetzung im wesentlichen derjenigen des Bluts des zu behandelnden Patienten entsprechen soll.

Es sind seit langem Verfahren bekannt, mit denen gebrauchsfertige Dialysierflüssigkeiten einstufig aus fertig in Kanistern angelieferten Flüssigkonzentraten hergestellt werden. Gleichermaßen sind Verfahren bekannt, mit denen Dialysierflüssigkeiten in einem Tank bettseitig (ebenso einstufig) aus einem Trockenkonzentrat und Wasser erzeugt werden.

Andererseits kann jedoch aber auch bettseitig zunächst in einer zweistufigen Methode ein Flüssigkonzentrat aus einem Trockenkonzentrat durch Vermischen mit Wasser hergestellt werden, aus dem anschließend durch weiteres Vermischen mit Wasser in einem vorbestimmten Mischungsverhältnis die gewünschte Dialysierflüssigkeit hergestellt wird.

Ein Verfahren der letztgenannten Art ist in der DE 32 12 230 beschrieben, bei dem zunächst ein Trockenkonzentrat in einer Mischkammer vorgelegt wird, die eingangsseitig mit einer Wasserquelle und ausgangsseitig mit einem Dialysierflüssigkeitstank verbunden ist. Hierdurch kann ein individuell angepaßtes Flüssigkonzentrat und daraus eine fertige Dialysierflüssigkeit hergestellt werden. Diese Mischkammer hat im wesentlichen die Aufgabe, nicht nur die leicht löslichen Elektrolytsalze, wie Natriumchlorid, Kaliumchlorid oder Calciumchlorid, sondern auch das in Wasser schwerer lösliche Natriumbicarbonat nur in gelöster Form in die Ausgangsleitung zu überführen. Die Mischung erfolgt, wie bei Batch-Verfahren allgemein üblich, volumetrisch, d.h. eine vorbestimmte Menge Trockenkonzentrat wird mit einem vorbestimmten Volumen Wasser umgesetzt. Bei diesem Mischprinzip wird aus einem Trockenkonzentrat und Wasser ein Flüssigkonzentrat in einen ersten Behälter zumindest zeitweise kontinuierlich und bei einer weiteren Variante aus Flüssigkeit und Wasser eine fertige Dialysierflüssigkeit in dem gleichen oder einem weiteren Behälter zubereitet, wobei dieser Behälter die gesamte Dialysierflüssigkeitsmenge für eine gesamte Behandlung enthält.

In der DE 34 43 911 ist ein Verfahren beschrieben, bei dem ein Flüssigkonzentrat aus Trockenkonzentrat und Wasser in einem Mischbehälter zubereitet wird, der über eine Eingangsleitung und über eine Ausgangsleitung verfügt. Ausgangsseitig kann dann Flüssigkonzentrat einem Mischpunkt zugeführt werden, dem über eine zweite Leitung Wasser in einer vorbestimmten Menge zugeführt wird. Üblicherweise wird in dem Mischbehälter ein Flüssigkonzentrat-Batch für eine gesamte Behandlung erzeugt, so daß im Anschluß daran eine fertige Dialysierflüssigkeit durch kontinuierliche Mischung des Flüssigkonzentrats mit Wasser hergestellt werden kann.

Aus US 4 386 634 ist ein weiteres Verfahren beschrieben, mit dem eine Dialysierflüssigkeit aus Trockenkonzentrat mittels eines zweistufigen Verfahrens hergestellt werden kann, wie es ebenfalls Gegenstand der Erfindung ist. Dabei wird ein Beutel als Mischgefäß für eine batchweise Fertigung von Flüssigkonzentrat benutzt, der während der Behandlung kontinuierlich zu einem Mischpunkt entleert wird, der ebenfalls mit Wasser zur Herstellung einer gebrauchsfertigen Dialysierflüssigkeit beaufschlagt wird. Die batchweise Herstellung von Flüssigkonzentrat hat zwar den Vorteil einer sicheren Mischung des Trockenkonzentrats mit Wasser, andererseits jedoch den Nachteil, daß hierdurch ein relativ großer Raumbedarf für das Konzentrat (8-12 l) notwendig ist, so daß hierdurch stabile Behälter oder aufwendige Lagerungs- und Halterungseinrichtungen notwendig sind. Beide Anordnungen sind dabei mit relativ hohen Kosten für die Desinfektion bzw. für die Bereitstellung verbunden.

Insofern hat man versucht (EP 278 100), Dialysierflüssigkeiten dadurch herzustellen, daß man festes Konzentrat relativ geringen Volumens in einer Kartusche vorgelegt hat, die über einen Wassereinlaß und über einen Flüssigkonzentratauslaß verfügt. Diese Kartusche ist mit einer Monosubstanz, üblicherweise Natriumbicarbonat gefüllt, das mit Wasser von der Kartuscheneingangsseite beaufschlagt wird, so daß sich beim Durchströmen der Flüssigkeit durch die Kartusche hindurch Bicarbonat langsam auflöst und ein Flüssigkonzentrat gebildet wird. Dieses Konzentrat wird durch den Auslaß einem Mischpunkt zugeführt, an dem es mit Wasser und weiteren Flüssigkonzentraten zu einer Dialysierflüssigkeit vorbestimmter Zusammensetzung verdünnt wird.

Dieses kontinuierlich arbeitende Verfahren hat den Vorteil des geringen Raumbedarfs für das Konzentrat und der guten Lagerfähigkeit des Trockenkonzentrats in der Kartusche im Vergleich zu einem in einem Kanister angelieferten Flüssigkonzentrat. Nachteilig an diesem Verfahren und dieser Anordnung sind, daß das Pulver stetig durchflossen werden muß, um die gewünschte Sättigung der Lösung durch Auflösen des Salzes einzustellen. So lösen sich bekanntlich etwa 100 g Natriumbicarbonat bei Raumtemperatur in 1 l Wasser auf. Diese Sättigung ist am Ausgang der Kartusche sicherzustellen, was jedoch durch das häufige Zusammenkleben des Pulvers und die gefürchtete Kanalbildung innerhalb der Kartusche bei ständigem Durchströmen des Wassers häufig nicht sichergestellt werden kann. Insofern ergeben sich aufgrund der unterschiedlichen Zusammensetzung der Flüssigkonzentrate Schwierigkeiten bei der Herstellung der fertigen Dialysierflüssigkeit, was insbesondere bei hohen Mischungsverhältnissen von Wasser zu Flüssigkonzentrat der Fall ist.

Des weiteren sind die Konnektoren von zwei Anschlüssen mit den bekannten Hygiene-und Verwechslungsproblemen behaftet.

Eine weitere Vorrichtung zur kontinuierlichen Herstellung von Flüssigkonzentrat ist in DE 41 39 165 beschrieben, bei der jedoch ebenfalls die vorstehend beschriebenen Kanalbildungs- und Hygiene- sowie Verwechslungsprobleme auftreten können.

Die EP 0 469 487 und die EP 0 575 970 beschreiben medizinische Verpackungseinheiten, die ein pulvefförmiges Konzentrat zur Herstellung von Dialysierflüssigkeit enthalten. Beide Verpackungseinheiten werden im Durchfluß betrieben. Daher weisen sie sowohl eine Zuleitung zum Zuführen von Wasser als auch eine Ableitung zum Abführen von Dialysierflüssigkeitskonzentrat auf. Ein besonderer Vorteil der aus der EP 0 575 970 bekannten Verpackungseinheit liegt darin, daß der Konzentratbehälter über nur einen einzigen Anschluß verfügt, der das Zuströmen von Wasser und das Abführen von verdünntem Konzentrat ermöglicht. Dieser Anschluß umfaßt jedoch nach wie vor zwei Schlauchleitungen zum Zu- und Abführen von Flüssigkeit, um den Behälter im Durchfluß betreiben zu können.

Der Erfindung liegt daher die Aufgabe zugrunde, eine Verpackungseinheit zur Verfügung zu stellen, mit der kontinuierlich bettseitig ein gesättigtes flüssiges Konzentrat ohne Kanalbildungs- oder Lagerungsprobleme hergestellt werden kann.

Gelöst wird diese Aufgabe zum einen durch die Zur-Verfügung-Stellung einer vorzugsweise flexiblen medizinischen Verpackungseinheit, die nur ein einziges Anschlußstück aufweist, so daß die Sterilitätsprobleme auf ein Minimum reduziert werden. Des gleichen ist der Inhalt der Verpackungseinheit derartig, daß portionsweise gesättigte Dialysatkonzentrate hergestellt werden können, d.h. während der Behandlung eines Patienten muß die Verpackungseinheit mehrmals mit frischem Wasser aufgefüllt werden, so daß jeweils batchweise Flüssigkonzentrate (Multi-Batch-Verfahren) hergestellt werden.

Da der vorzugsweise flexible Behälter nur über ein einziges Anschlußstück verfügt, weist er auch ein gemeinsames Leitungsstück für das zugeführte Wasser und das abzuführende Flüssigkonzentrat auf. Dieses Totvolumen befindet sich üblicherweise in dem zweiten Leitungsstück zwischen dem Verknüpfungspunkt und dem Anschlußstück des Behälters.

Die dem Behälter jeweils zugeführte Wassermenge reicht üblicherweise nicht aus, um auch nur einen erheblichen Teil des Bicarbonat-Konzentrats im Beutel zu lösen.

Üblicherweise werden nur etwa 10-20 % des Konzentrats im Beutel aufgelöst. So wird beispielsweise bei einem Beutel, der ein füllbares Innenvolumen von 1,5 l aufweist und mit etwa 650 g Natriumbicarbonat und etwa 1 l Wasser gefüllt ist, etwa 100 g Natriumbicarbonat bei Raumtemperatur unter Bildung einer gesättigten Lösung aufgelöst, so daß mindestens weitere fünf Füll- und Entleerungsoperationen durchgeführt werden können, ohne daß die Gefahr besteht, die Sättigungskonzentration nicht mehr zu erreichen.

Damit auch tatsächlich das zugeführte Wasser durch Vermischung mit dem pulverförmigen Konzentrat in eine gesättigte Lösung umgewandelt wird, wird vorteilhafterweise die im Beutel befindliche Lösung in Bewegung gehalten. Dies kann beispielsweise dadurch erfolgen, daß der Beutel geschüttelt oder mit Ultraschall behandelt wird, so daß eine innige Durchmischung erfolgt.

Der flexible Behälter, vorzugsweise ein Beutel, der zur Aufnahme des Trockenkonzentrats dient, wird gemäß einer ersten Ausführungsform von unten mit Wasser beaufschlagt, d.h. das Anschlußstück befindet sich bei aufgehängtem Beutel am unteren Ende. Um eine Verschleppung von Trockenkonzentrat nach dem Mischen mit Wasser zu verhindern, werden vorteilhafterweise Abtrenneinrichtungen im Behälter verwendet. So ist beispielsweise die Anschlußöffnung des Behälters mit einer halbdurchlässigen Membran verschlossen, die Wasser und Flüssigkonzentrat, nicht jedoch das Trockenkonzentrat aufgrund seiner Partikelgröße (ca. 0,3 mm und größer) durchläßt. Wird bei dieser Ausführungsform Wasser zugeführt, so findet bereits im Bodenbereich eine innige Durchmischung des Trockenkonzentrats mit Wasser statt, so daß bei dem Durchströmen des Pulvers zur Auffüllung des Behälters sich sofort gesättigtes Konzentrat bildet.

Gemäß einer weiteren Ausführungsform kann der Behälter in umgekehrter Orientierung benutzt werden, d.h. das Anschlußstück befindet sich in der Gebrauchslage oben. In einem solchen Fall wird ein Steigrohr in den Beutel integriert, das bis an die tiefste Stelle des Behälters geführt ist. Dabei ist das Ende des Steigrohrs entweder wiederum mit einer halbdurchlässigen Membran oder aber mit einem Rückschlagventil gesperrt, das nur den Zufluß, nicht jedoch aber den Abfluß von Flüssigkonzentrat zuläßt. Im letzteren Fall befindet sich eine zweite Öffnung benachbart zum Anschlußstück im Steigrohr, das durch ein zweites Rückschlagventil in umgekehrter Richtung zum ersten Rückschlagventil versperrt ist, so daß nur die Abförderung möglich ist.

Die Form und die Anordnung des Konzentrat-Behälters kann weitgehend frei gewählt werden, solange ein ausreichender Kontakt des Wassers mit dem Trockenkonzentrat in der Füllphase gewährleistet ist.

Es zeigen
Fig. 1 schematisch eine erste Ausführungsform eines Trockenkonzentiatbehälters,
Fig. 2 schematisch eine zweite Ausführungsform eines Trockenkonzentrat-Beutels,
Fig. 3 schematisch eine dritte Ausführungsform eines Trockenkonzentrat-Beutels,
Fig. 4 schematisch eine vierte Ausführungsform eines Trockenkonzentrat-Beutels und
Fig. 5 schematisch eine fünfte Ausrührungsform eines Trockenkonzentrat-Beutels.

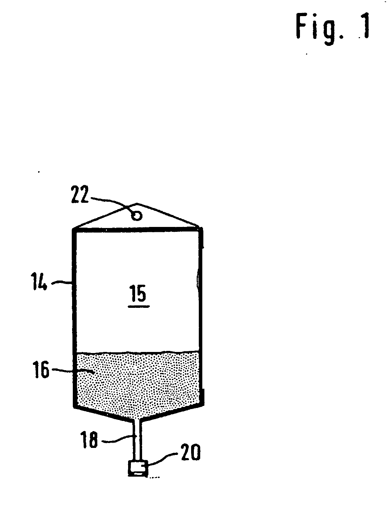

Der in Fig. 1 gezeigte Behälter 14 ist flexibel ausgeführt, vorteilhafterweise in Beutelform. Hierzu werden üblicherweise Kunststoffolien eingesetzt, um derartige Beutel herzustellen.

Der Behälter 14 ist mit einem Trockenkonzentrat 16 teilweise gefüllt, das üblicherweise eine Partikelgröße von 0,3 mm und mehr aufweist. Als Trockenkonzentrat können sämtliche bei der Dialyse eingesetzten Elektrolyte in Frage kommen, vorzugsweise das schwer wasserlösliche Natriumbicarbonat. Dieses Trockenkonzentrat liegt in dem Beutel in einer solchen Menge vor, daß mindestens eine Hämodialyse-Behandlung durchgeführt werden kann.

Wird Natriumbicarbonat in dem Behälter 14 vorgelegt, so werden vorteilhafterweise sämtliche anderen Konzentrate (Natriumchlorid, Kaliumchlorid, Calciumchlorid) in einem weiteren, nicht gezeigten Behälter entweder in Trockenform oder in Flüssigkonzentratform vorgelegt.

Üblicherweise enthält der Behälter ca. 500-1 000 g Natriumbicarbonat (je nach herzustellender Gesamtflüssigkeitsmenge). Weiterhin beträgt das Innenvolumen des Behälter 14 das 2-4fache des Ausgangsvolumens des pulverförmigen Natriumbicarbonats bzw. der anderen Trockenkonzentrate.

Der Behälter 14 verfügt über ein einziges rohrförmiges Anschlußstück 18, dessen Ende vorteilhafterweise ein Verbindungsstück 20 aufweist.

Des weiteren ist gemäß der in Fig. 1 gezeigten Ausführungsform auf der gegenüberliegenden Seite des Behälters 14 eine Aufhängeeinrichtung 22, üblicherweise eine Öse vorgesehen, mit der der Behälter 14 an einem nicht gezeigten Stativ aufgehängt werden kann.

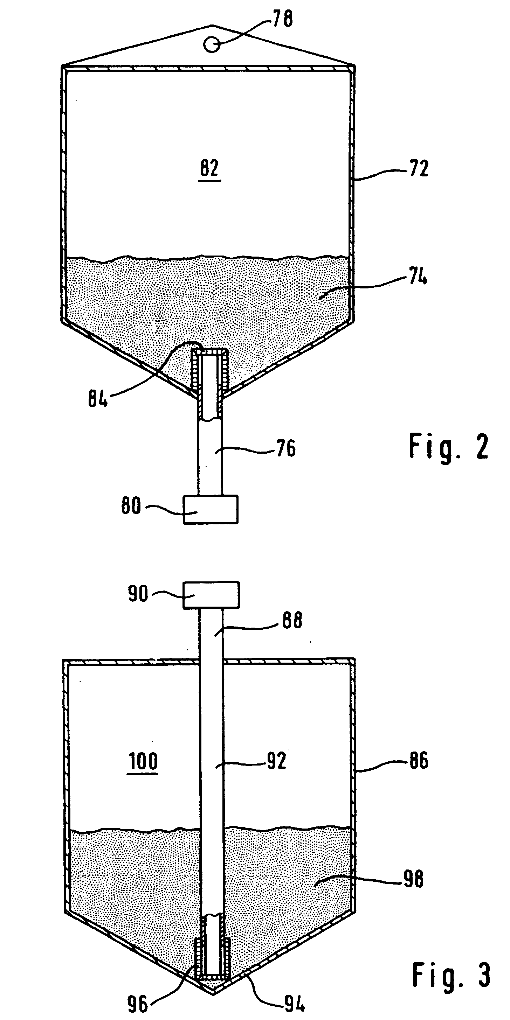

In Fig. 2 ist eine zweite Ausführungsform eines erfindungsgemäßen Behälters 14, wie er bereits in Fig. 1 gezeigt ist, in Form eines Beutels 72 gezeigt, der mit einem trockenen Elektrolytkonzentrat, vorzugsweise Natriumbicarbonat in einer solchen Menge gefüllt ist, daß die Behandlung eines Patienten während einer Sitzung gewährleistet ist. Üblicherweise liegt diese Menge zwischen 500 und 800 g Bicarbonat, das vorteilhafterweise eine mittlere Körnung von 0,5 mm und darüber aufweist. Diese Konzentratschicht ist mit 74 bezeichnet.

Bei dem in Fig. 2 gezeigten Beutel 72 handelt es sich um ein Hängesystem, bei dem der rohrförmige Anschußstutzen 76 in der Gebrauchslage unten angeordnet ist. Gegenüber dem Anschlußstutzen 76 befindet sich eine Aufhängeöse 78 im Rand des Beutels 72.

Des weiteren ist der Anschlußstutzen 76 mit einem Konnektorstück 80 versehen, wie es bereits vorstehend erwähnt worden ist. Durch den Anschlußstutzen 76 hindurch besteht eine Strömungsverbindung mit dem Innenraum 82 des Beutels, der üblicherweise zu 1/3-1/4 seines Volumens mit dem Konzentrat gefüllt ist. Der Rest des Innenraums 82 kann mittels Wasser bzw. gesättigter Konzentratlösung aufgefüllt sein.

Das im Innenraum 82 liegende Ende des Anschlußstutzens 76 ist mit einer halbdurchlässigen Membranschicht oder Filterschicht 84 verschlossen, die die Eigenschaft hat, einerseits zuströmendes Wasser und abströmende Konzentratlösung durchzulassen, andererseits Konzentratpulver oder -granulat am Abströmen durch den Anschlußstutzen 76 hindurch zu hindern. Ein Filter hält beispielsweise sämtliche Partikel zurück, die eine Korngröße von mehr als 0,3 µm aufweisen. Kleinere Partikel können dagegen im Konzentratstrom mitgerissen werden und lösen sich spätestens in der Einrichtung 40 auf. Ihre Konzentration ist derart gering, daß sie keine Nennenswerte Änderung der Zusammensetzung der Dialysierflüssigkeit nach sich ziehen.

In Fig. 3 ist eine weitgehend der Ausführungsform von Fig. 2 ähnliche Form gezeigt, wobei es sich hier um einen Beutel 86 handelt, bei dem der Anschlußstutzen 88 in der Gebrauchslage oben ist. Dabei kann das Konnektorstück 90 nicht nur zum Konnektieren, sondern auch zum Befestigen des Beutels 86 an dem nicht gezeigten Dialysegerät dienen.

Der Beutel 86 weist dabei ein Tauchrohr 92 auf, das sich im Anschluß an den Anschlußstutzen 88 bis zum Bodenbereich 94 erstreckt, der gemäß der in Fig. 3 gezeigten Ausführungsform vorteilhafterweise konisch nach unten spitz zulaufend verläuft.

Das Ende des Tauchrohrs 92 ist mit der gleichen Membran- oder Filterschicht 96 verschlossen, wie er mit 84 bei der Ausführungsform von Fig. 2 gezeigt ist.

Diese Filterschicht 96 befindet sich in der pulverförmigen Konzentratschicht 98, die der Konzentratschicht 74 gleicht.

Die Ausführungsformen von Fig. 2 und 3 haben den Vorteil, daß die in die Beutel 72 und 86 beförderte Flüssigkeit zweimal die Konzentratschicht 74 und 98 durchqueren muß. So fließt das Wasser zunächst beim Zufördern durch die Konzentratschicht hindurch und sättigt sich weitgehend bzw. vollständig mit Natriumbicarbonat. Wird anschließend Lösung aus dem Innenraum 82 bzw. 100 des Beutels 86 abgepumpt, so muß die aus dem Innenraum nachstehende Lösung nochmals durch die Konzentratschicht hindurch und löst, sofern sie nicht bereits gesättigt ist, pulverförmiges Konzentratsalz bis zu ihrer Sättigung auf.

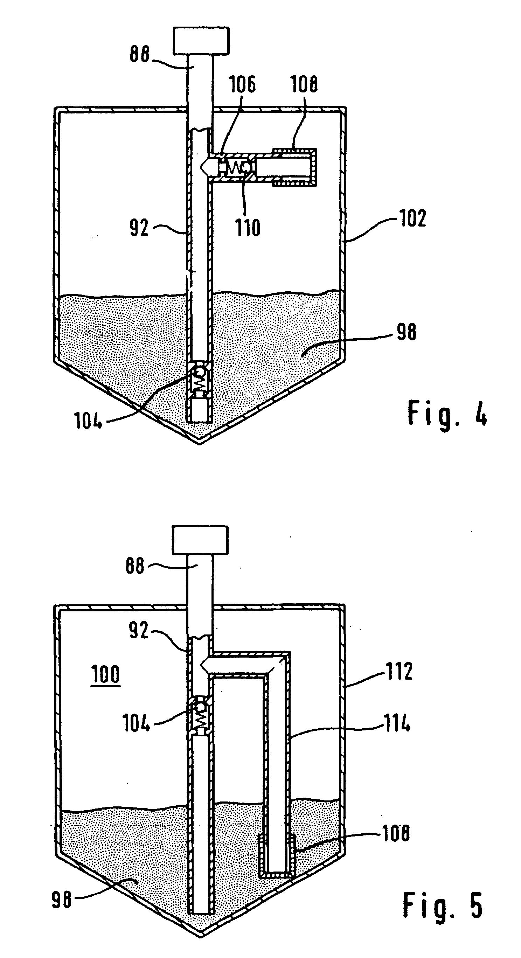

In Fig. 4 ist eine vierte Ausführungsform eines Beutels 102 gezeigt, der im wesentlichen der Ausführungsform gemäß Fig. 3 entspricht, so daß auf die Ausführungsform gemäß Fig. 3 mit ihren Bezugszeichen Bezug genommen wird. Bei der in Fig. 4 gezeigten Ausführungsform wird jedoch nur der Zufluß von Wasser durch das Tauchrohr 92 bis zu seinem Ende zugelassen, denn ein Einwegventil 104 sperrt den Rückfluß in Richtung Anschlußstück 88. Infolgedessen ist benachbart zum Anschlußstück 88 im Innenraum 100 ein Abflußstutzen bzw. eine Öffnung 106 am Tauchrohr 92 vorgesehen, durch das Flüssigkonzentrat abgezogen werden kann. Gemäß einer ersten Ausführungsform ist das Ende des Abflußstutzens 106 mit einer Membran bzw. Filterschicht 108 verschlossen, die den vorstehend genannten Filterschichten 84 und 96 entspricht. Gemäß einer anderen Ausführungsform ist in dem Abflußstutzen ein zweites Rückschlagventil 110 vorgesehen, das nur den Abfluß in Richtung Anschlußstutzen 88 zuläßt, ansonsten aber den Zufluß von Wasser sperrt.

Eine weitere Ausführungsform eines Beutels 112 ist in Fig. 5 dargestellt, die weitgehend der Ausführungsform gemäß Fig. 4 bzw. Fig. 3 entspricht, so daß auch hier wieder die gleichen Bezugszeichen für gleiche Teile eingesetzt werden.

Anstelle des Abflußstutzens 106 ist ein zweites Tauchrohr 114 vorgesehen, das mit der Membran 108 verschlossen ist. Dieses Tauchrohr durchsetzt die Konzentratschicht 98 und ist an ihrem Ende wiederum mit der Filterschicht 108 verschlossen. Andererseits zweigt sie benachbart vom Anschlußstutzen 88 innerhalb des Beutels 112 vom Rückschlagventil 104 im ersten Tauchrohr 92 ab.

Dabei läßt das erste Rückschlagventil 104 den Zufluß von Wasser durch den Innenraum 100 und die Konzentratschicht 98 zu, sperrt jedoch den Rückfluß. Dieser erfolgt dann durch die Filterschicht 108, das zweite Tauchrohr 114, den oberen Teil des ersten Tauchrohrs 92 und den Anschlußstutzen 88.

Die Beutel 72,86,102 und 112 sind jeweils eine selbständige Handelseinheit und können bei Bedarf an eine Einrichtung angeschlossen werden. Dabei ist unter Beutel nicht nur der übliche Kunststoffbeutel, der vorzugsweise aus einem klaren durchsichtigen Material besteht, sondern auch ein Behälter mit festen Wänden zu verstehen, der dann über ein Belüftungsmittel, üblicherweise eine hydrophobe Membran, die Luft, nicht jedoch Wasser durchläßt, verfügt. Ein solcher Behälter kann ebenfalls aus einem klaren Kunststoffmaterial oder aber aus Glas bestehen. Solche Gefäße mit festen Wänden können dann zweckmäßig sein, wenn Konzentrate aufgelöst werden, bei denen kein Gas, wie bei Bicarbonat durch Disproportionierung von Bicarbonat in Carbonat und CO₂, entsteht. Insofern ist für die Auflösung von Bicarbonat ein Behälter mit festen Wänden nicht bevorzugt.

## Patentansprüche

1. Medizinische Verpackungseinheit für die Hämodialyse zur Herstellung eines Dialysierflüssigkeitskonzentrats mit einem Behälter (14), in dem pulverförmiges Salz in einer für eine Dialysebehandlung ausreichenden Menge enthalten ist, wobei das Volumen des Innenraums (15) des Behälters und die Menge des pulverförmigen Salzes jeweils so bemessen sind, daß bei völliger Füllung des Behälters mit Wasser bei Raumtemperatur nur ein Teil des Salzes gelöst ist und die Sättigungskonzentration erreicht wird, **dadurch gekennzeichnet, daß** die medizinische Verpackungseinheit zur portionsweisen Herstellung von gesättigten Dialysierflüssigkeitskonzentratmengen nur ein einziges Anschlußstück mit nur einem Lumen sowohl für die Zuleitung als auch Ableitung von Flüssigkeit aufweist.

2. Verpackungseinheit nach Anspruch 1, **dadurch gekennzeichnet, daß** das Salzkonzentrat Natriumbicarbonat ist.

3. Verpackungseinheit nach Anspruch 1 oder 2, **dadurch gekennzeichnet, daß** der Behälter (14) ein flexibler Kunststoffbeutel ist.

4. Verpackungseinheit nach einem der Ansprüche 1 bis 3, **dadurch gekennzeichnet, daß** benachbart zum Anschlußstück (76) im Innenraum (82) des Behälters eine Filterschicht (84) vorgesehen ist, durch die hindurch eine Strömungsverbindung vom Anschlußstück (76) zum Innenraum (82) geschaffen wird.

5. Verpackungseinheit nach einem der Ansprüche 1 bis 3, **dadurch gekennzeichnet, daß** sich ein Tauchrohr (92) vom Anschlußstück (88) durch den Innenraum (100) des Behälters (86,102,112) bis zum Bodenbereich (94) erstreckt.

6. Verpackungseinheit nach Anspruch 5, **dadurch gekennzeichnet, daß** das im Bodenbereich (94) befindliche Ende des Tauchrohrs (92) mit einer Filterschicht (96) verschlossen ist.

7. Verpackungseinheit nach Anspruch 5, **dadurch gekennzeichnet, daß** im Tauchrohr (92) ein erstes Rückschlagventil (104) vorgesehen ist, das nur den Fluß in Richtung Bodenbereich (94) des Behälters (102,112) zuläßt, und benachbart zum Anschlußstück (88) das Tauchrohr (92) eine Öffnung oder einen Abflußstutzen (106) aufweist, wobei an der Öffnung oder am Abflußstutzen (106) ein zweites Rückschlagventil (110), das nur die Abströmung von Flüssigkeit in Richtung Anschlußstutzen (88) freigibt, und/oder eine weitere Filterschicht (108) angeordnet sind.

8. Verpackungseinheit nach Anspruch 7, **dadurch gekennzeichnet, daß** sich der Abflußstutzen (106,114) bis in die Konzentratschicht (98) erstreckt.

## Claims

1. Medical pack for haemodialysis for the preparation of a dialysate concentrate with a container (14) in which salt in powder form in a quantity adequate for one dialysis treatment is contained, whereby the capacity of the interior (15) of the container and the amount of salt in powder form are sized in each case so that with the container completely filled with water at room temperature only part of the salt dissolves and saturation concentration is reached, **characterised in that** the medical pack for the preparation of saturated amounts of dialysate concentrate in portions has only a single connecting piece with only one lumen both for the supply and drainage of the fluid.

2. Pack in accordance with claim 1, **characterised in that** the salt concentrate is sodium bicarbonate

3. Pack in accordance with claim I or 2, **characterised in that** the container (14) is a flexible plastic bag.

4. Pack in accordance with one of claims 1 to 3, **characterised in that** a filter layer (84) is provided adjacent to the connecting piece (76) in the interior (82) of the container, through which a flow is created from the connection piece (76) to the interior (82).

5. Pack in accordance with one of claims 1 to 3, **characterised in that** an immersion tube (92) extends from the connecting piece (88) through the interior (100) of the container (86, 102, 112) to the bottom area (94).

6. Pack in accordance with claim 5, **characterised in that** the end of the immersion tube (92) at the bottom area (94) is sealed by a filter layer (96).

7. Pack in accordance with claim 5, **characterised in that** in the immersion tube (92) a first check valve (104) is provided that permits flow only in the direction of the bottom area (94) of the container (102, 112) and that adjacent to the connecting piece (88) the immersion tube (92) has an opening or a drain tube (106) with a second check valve (110) being provided at the opening or on the drain tube (106) that enables only draining of the fluid in the direction of the connecting tube (88), and/or a further filter layer (108).

8. Pack in accordance with claim 7, **characterised in that** the drain tube (106, 114) extends into the concentrate layer (98).

## Revendications

1. Unité de conditionnement médical pour l'hémodialyse pour la préparation d'un concentré de liquide de dialyse dans un récipient (14), dans lequel le sel pulvérulent est contenu dans une quantité suffisante pour un traitement par dialyse, le volume de l'espace interne (15) du conteneur et la quantité du sel pulvérulent sont calculés de façon telle que, pour un remplissage complet du conteneur par l'eau à température ambiante, il ne se dissolve qu'une partie du sel et on atteint la concentration de saturation, **caractérisée en ce que** l'unité de conditionnement médical pour la préparation de portions de quantités de liquide de dialyse saturé, ne présente qu'une seule pièce de raccordement avec une seule lumière aussi bien pour l'apport que pour l'évacuation du liquide.

2. Unité de conditionnement selon la revendication 1, **caractérisée en ce que** le concentré de sel est le bicarbonate de sodium.

3. Unité de conditionnement selon la revendication 1 ou 2, **caractérisée en ce que** le conteneur (14) est une poche en matière synthétique souple.

4. Unité de conditionnement selon l'une des revendications 1 à 3, **caractérisée en ce qu'**on prévoit dans l'espace interne (82) du conteneur, adjacente à la pièce de raccordement (76), une couche filtrante (84), à travers laquelle est créé une liaison par écoulement depuis la pièce de raccordement (76) à l'espace interne (82).

5. Unité de conditionnement selon l'une des revendications 1 à 3, **caractérisée en ce qu'**un tube plongeant (92) s'étend depuis la pièce de raccordement (88) à travers l'espace interne (100) du conteneur (86, 102, 112) jusqu'à la région du fond (94).

6. Unité de conditionnement selon la revendication 5, **caractérisée en ce que** l'extrémité du tube plongeant (92), qui se trouve dans la région du fond (94), est fermée par une couche filtrante (96).

7. Unité de conditionnement selon la revendication 5, **caractérisée en ce qu'**on prévoit une soupape de retenue (104) dans le tube plongeant (92), qui ne permet que l'écoulement dans la direction de la région du fond (94) du conteneur (102, 112) et que le tube plongeant (92) adjacent de la pièce de raccordement (88) présente un orifice ou une rallonge de déchargeoir (106), où sur l'orifice ou sur la rallonge de déchargeoir (106) est disposée une seconde soupape de retenue (110), qui ne permet que l'écoulement de liquide dans la direction de la rallonge de déchargeoir (88), et/ou une autre couche filtrante (108).

8. Unité de conditionnement selon la revendication 7, **caractérisée en ce que** la rallonge de déchargeoir (106, 114). s'étend jusqu'à la couche de concentré (98).
